# EUROPÄISCHE PATENTSCHRIFT

(11) **EP 3 630 296 B1**
(45) Veröffentlichungstag und Bekanntmachung des Hinweises auf die Patenterteilung: **02.08.2023**
(21) Anmeldenummer: 18717065.9
(22) Anmeldetag: 13.04.2018
(51) Int. Cl.: A61K 8/81, A61Q 5/00, A61Q 19/00

(54) **VERWENDUNG BESTIMMTER POLYMERE ZUR HERBEIFÜHRUNG EINES ANTI-POLLUTION-EFFEKTS**
USE OF CERTAIN POLYMERS TO INVOKE AN ANTI-POLLUTION EFFECT
UTILISATION DE POLYMÈRES DÉTERMINÉS DESTINÉ À L'ÉTABLISSEMENT D'UN EFFET ANTI-POLLUTION

(30) Priorität: 23.05.2017 EP 17172546
(43) Veröffentlichungstag der Anmeldung: 08.04.2020
(73) Patentinhaber: BASF SE, 67056 Ludwigshafen am Rhein (DE)
(72) Erfinder: MEHLING, Annette, 40589 Düsseldorf-Holthausen (DE); RIEDEL, Heidi, 40589 Düsseldorf-Holthausen (DE); KOCH, Jan Peter, 40589 Düsseldorf-Holthausen (DE); GONDEK, Helga, 40589 Düsseldorf-Holthausen (DE); HOESSEL, Peter, 67105 Schifferstadt (DE)
(74) Vertreter: BASF IP Association
(86) Internationale Anmeldenummer: PCT/EP2018/059581
(87) Internationale Veröffentlichungsnummer: WO 2018/215136

(56) Entgegenhaltungen:
- WO-A1-2017/057944
- WO-A1-2017/074125
- FR-A1- 2 836 633
- KR-B1- 101 653 755
- US-A1- 2002 051 797

## Beschreibung

Die vorliegende Erfindung betrifft die Verwendung eines Polymers oder eines kosmetischen Produkts enthaltend dieses Polymer und zusätzlich andere, bekannte kosmetischen Inhaltsstoffe zur Herbeiführung eines Anti-Pollution-Effekts für menschliche Haut oder für menschliche Haare, insbesondere zum Schutz menschlicher Haut oder menschlicher Haare vor Staub, wobei das Polymer ein kationisches, anionisches oder nichtionisches Polymer ist, und wobei das Polymer eine Wasserlöslichkeit von mindestens 0,01 g Polymer in 100 g Wasser bei 20° C bei mindestens einem pH-Wert im Bereich zwischen 4 und 9 hat.

Die Umgebungsluft der Menschen ist vielfach durch Luftverschmutzung beeinträchtigt. Ein Bestandteil der Luftverschmutzung sind partikuläre Substanzen, darunter Staub und Sand, darunter auch feine Stäube, darunter Feinstaub.

Aus kosmetischer Sicht ist der Schutz der menschlichen Haut oder der menschlichen Haare gegen Luftverschmutzung, insbesondere gegen die Ablagerung von Staub aus der Luft wünschenswert, unter anderem weil das Erscheinungsbild der Haut oder der Haare durch die Ablagerung von Staub beeinträchtigt werden kann, z. B. kann die Haut dann grau oder gräulich erscheinen. Das Anhaften der Partikel und Substanzen auf den Partikeln können auch zu anderen Effekten führen, wie z.B. zu vorzeitiger Hautalterung (z.B. zu Altersflecken), zu Hautoberflächenveränderungen wie trockene, und/oder raue Haut, zu inhomogenen Pigmentierungen (Englisch: uneven skin tone), zu verminderter Strahlkraft (Englisch: radiance) zu einer Erhöhung des transepidermalen Wasserverlusts, sowie zur Verminderung der Hautelastizität, Haut-Weichheit und Haut-Geschmeidigkeit. Auch Veränderungen am Haar wie eine Erhöhung der Brüchigkeit und Rauheit können hervorgerufen werden. Auch die Empfindlichkeit der Haut und/oder der Kopfhaut können gesteigert werden.

Es besteht daher ein Bedarf nach kosmetischen Produkten, die die Haut oder die Haare gegen Luftverschmutzung, insbesondere gegen partikuläre Substanzen, insbesondere Sand oder Staub, insbesondere gegen feine Stäube schützen. Ein derartiger Schutz wird in der Kosmetik auch als Anti-Pollution-Effekt für die menschliche Haut oder für die menschlichen Haare bezeichnet. Mit anderen Worten besteht also ein Bedarf nach kosmetischen Produkten, die einen Anti-Pollution-Effekt für die menschliche Haut oder für die menschlichen Haare bewirken.

US 4 913 897 offenbart ein hydrophiles Hydrogel als Schutzfilm vor Verschmutzung und vor toxischen Substanzen. Es werden Hydrogele aus Polyurethanen, aus Polyacrylnitril und aus Copolymeren des Acrylnitrils und aus Polyvinylactat offenbart. Unter Verschmutzung werden toxische Substanzen und infektiöse Substanzen verstanden.

DE 31 47 024 A1 offenbart eine filmbildende Matrix (offenbart werden Polyethylenoxid, Polysaccharide, Polyvinylalkohol) mit anorganischen Teilchen (Talkum, Glimmer, Kreide) mit einem Durchmesser von 10 bis 200 Mikrometer zum Schutz vor Verschmutzung. Unter Verschmutzung werden giftige Gase und Flüssigkeiten verstanden.

EP 1 684 710 A1 offenbart ein Ölgel mit einem Polymer (eine wasserabweisende Substanz mit einem wasserabsorbierenden Puder).

EP 0 884 047 A1 offenbart den Einsatz von Polyamino-Polymeren zum Schutz vor lichtinduzierter Peroxidation von Lipiden und Proteinen.

EP 1 157 683 A1 offenbart Fasern (Polyester, Polyurethan, Acryl, Baumwolle und andere) in kosmetischen Zubereitungen zum Schutz gegen Luftverschmutzung, Ozon, Kohlenmonooxid, Stickstoffoxide und Schwermetalle. Die offenbarten Fasern sind nicht wasserlöslich.

EP 0 577 718 A1 offenbart Sphingolipide (Ceramide und andere) in kosmetischen Zubereitungen zum Schutz gegen Verschmutzung (Luftverschmutzung, Ozon, Kohlenmonooxid, Stickstoffoxide, Schwermetalle).

EP 1 447 074 A1 offenbart den Einsatz von quervernetzten polymeren Nanopartikeln mit einem Durchmesser von 1-10 nm in kosmetischen Produkten (z. B. Skin Care Produkten).

KR 101 653 755 offenbart die Verwendung eines Silikon-Acrylat-Copolymers für anti-pollution Zwecke.

FR 2 836 633 offenbart die Verwendung einer Zusammensetzung enthaltend ein Poly-N-vinylpyrrolidon-Homopolymer für anti-pollution Zwecke.

Ebenso kannte der Fachmann kosmetische Mittel zum Schutz gegen Verschmutzung aus WO 2017/057944 A1 und WO 2017/074125 A1.

Der vorliegenden Erfindung liegt die Aufgabe zugrunde, für die kosmetische Anwendung geeignete Substanzen bereitzustellen, die einen Anti-Pollution-Effekt für die menschliche Haut oder für die menschlichen Haare bewirken, das heißt, für die kosmetische Anwendung geeignete Substanzen bereitzustellen, die die Haut oder die Haare gegen Luftverschmutzung, insbesondere gegen Staub, insbesondere gegen Feinstaub schützen, bevorzugt dadurch, dass diese Substanzen die Adhäsion derjenigen Stoffe, die die Luftverschmutzung ausmachen, insbesondere Staub, insbesondere Feinstaub, auf der Haut oder den Haaren vermindern.

Diese Aufgabe wird gelöst durch die Verwendung gemäß Patentanspruch 1. Diese Verwendung gemäß Patentanspruch 1 ist ein Gegenstand der vorliegenden Erfindung. Die Unteransprüche sind bevorzugte Ausführungsformen der vorliegenden Erfindung.

Die Glasübergangstemperatur Tg ist dabei so zu bestimmen, wie im Abschnitt "Beispiele" beschrieben.

Einige der erfindungsgemäßen Polymer haben den weiteren Vorteil, dass sie die Abwaschbarkeit derjenigen Stoffe, die die Luftverschmutzung ausmachen, insbesondere Staub und auch Sand, insbesondere Feinstaub, mit Wasser verbessern.

Weitere Ausführungsformen der vorliegenden Erfindung sind gegeben durch die Verwendungen gemäß der Patentansprüche, wobei jeweils die Verwendung zur Herbeiführung eines Anti-Pollution-Effekts präzisiert wird auf die Verwendung zur Verminderung der Adhäsion derjenigen Stoffe, die die Luftverschmutzung ausmachen, insbesondere Staub, insbesondere Feinstaub, auf der Haut.

Weitere Ausführungsformen der vorliegenden Erfindung sind gegeben durch die Verwendungen gemäß der Patentansprüche, wobei jeweils die Verwendung zur Herbeiführung eines Anti-Pollution-Effekts präzisiert wird auf die Verwendung zur Verbesserung der Abwaschbarkeit derjenigen Stoffe, die die Luftverschmutzung ausmachen, insbesondere Staub, insbesondere Feinstaub, mit Wasser.

Ein weiterer Gegenstand der vorliegenden Erfindung und weitere bevorzugte Ausführungsformen dieses Gegenstandes sind ein Verfahren zur Herbeiführung eines Anti-Pollution-Effekts für menschliche Haut oder für menschliche Haare, insbesondere zum Schutz menschlicher Haut oder menschlicher Haare vor Staub, insbesondere vor Feinstaub, umfassend das Aufbringen des Polymers wie definiert in einem der Patentansprüche - oder das Aufbringen eines kosmetischen Produkts enthaltend das Polymers wie definiert in einem der Patentansprüche und zusätzlich enthaltend andere, bekannte kosmetischen Inhaltsstoffe - auf solche menschliche Haut oder solche menschlichen Haare, die eines Anti-Pollution-Effekts, insbesondere des Schutzes vor Staub, insbesondere des Schutzes vor Feinstaub, bedürfen.

Die anderen, bekannten kosmetischen Inhaltsstoffe, welche neben dem genannten Polymer in dem genannten kosmetischen Produkt enthalten sind, können sein Wasser, Tenside, Emulgatoren, anorganische oder organische Füllstoffe, Sensorikadditive (Substanzen deren Zugabe die sensorischen Eigenschaften modulieren sollen, z.B. Silikone, Puderrohstoffe, Elastomere, Actives (z.B. Menthol), etc.), Pigmente, Lichtschutzfilter, feuchtigkeitsspendende Stoffe (z. B. Polyole, insbesondere Glyzerin, Harnstoff), wasserlösliche oder fettlösliche Wirkstoffe, Additive (z. B. Konservierungsmittel, Duftstoffe, Komplexbildner oder Neutralisationsmittel), Konsistenzgeber (z. B. Fettalkohole, Glyceryl-Fettsäureester).

Das genannte kosmetischen Produkt enthaltend das genannte Polymer und andere, bekannte kosmetische Inhaltsstoffe kann insbesondere eine wässrige Zubereitung sein, zum Beispiel eine wässrige Lösung oder ein wässriges Gel (Hydrogel genannt), wobei diese Zubereitung bevorzugt 0 bis max. 20 Gew.-%, bevorzugt 0 - max. 10%, bevorzugt 0-5 Gew.-%, besonders bevorzugt 0 - max. 2,5 Gew.-% hydrophobe Bestandteile enthält. Unter hydrophoben Bestandteilen werden Öle, Fette, und Wachse verstanden.

Das genannte kosmetischen Produkt enthaltend das genannte Polymer und andere, bekannte kosmetische Inhaltsstoffe kann insbesondere sein ein halbfestes Gel, ein wässriges Gel, ein Pumpspray, ein Aerosolspray, eine Emulsion, wie z. B. eine Creme oder eine Lotion.

Das genannte kosmetische Produkt enthaltend das genannte Polymer und andere, bekannte kosmetische Inhaltsstoffe kann ein solches sein, das über Pinsel- / Schwamm- / Metall/Kunststoffapplikatoren, Tücher (z.B. wipes), Sprayanwendungen oder über die Hand appliziert werden kann.

Das genannte kosmetischen Produkt enthaltend das genannte Polymer und andere, bekannte kosmetische Inhaltsstoffe kann ein solches sein, das als Hautpflegeprodukt zum Schutz der Haut vor partikulären Substanzen und zur Pflege der Haut eingesetzt werden kann.

Das genannte kosmetischen Produkt enthaltend das genannte Polymer und andere, bekannte kosmetische Inhaltsstoffe kann ein solches sein, das als additiver "Top Coat" eingesetzt werden kann, der nach Applikation eines Hautpflegeproduktes (wie Tagescreme, Sonnenschutzcreme, Make-up, Foundation etc.) appliziert wird, und hierdurch insbesondere die Adhäsion partikulärer Substanzen (Staub) vermindert und ggf. deren Entfernbarkeit verbessert.

Das genannte kosmetischen Produkt enthaltend das genannte Polymer und andere, bekannte kosmetische Inhaltsstoffe kann ein solches sein, das als "Base Coat" eingesetzt werden kann, der vor der Applikation eines Hautpflegeproduktes (wie Tagescreme, Sonnenschutzcreme, Make-up, Foundation etc.) appliziert wird, und hierdurch insbesondere die Adhäsion partikulärer Substanzen (Staub) vermindert und ggf. deren Entfernbarkeit verbessert.

Die anderen, bekannten kosmetischen Inhaltsstoffe, welche neben dem genannten Polymer in dem genannten kosmetischen Produkt enthalten sein können (im Folgenden wird das Produkt auch Zubereitung genannt), können ausgewählt sein aus einem oder mehreren der im Folgenden beschriebenen Inhaltsstoffe.

Die kosmetischen Zubereitungen können Formulierungen zur Körperpflege sein, z. B. eine Körpermilch, Cremes, Lotionen, sprühbare Emulsionen, Produkte zur Eliminierung des Körpergeruchs etc. Die erfindungsgemäßen Polymere lassen sich auch in tensidhaltigen Formulierungen wie z. B. Schaum- und Duschbädern, Haarshampoos und Pflegespülungen einsetzen. Je nach Applikationszweck enthalten die kosmetischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Alle nun folgenden Ausführungen bis zum Beginn des Abschnitts "Beispiele" beschreiben lediglich besondere Ausführungsformen der vorliegenden Erfindung und keinesfalls zwingende Merkmale der vorliegenden Erfindung.

### Polyole und hautbefeuchtende Wirkstoffe

Diese können insbesondere sein Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin. Neben den Polyolen können weitere hautbefeuchtende Wirkstoffe wie Urea, Hyaluronsäure, Glyceryl Glucosid, Serine und Weitere zum Einsatz kommen

### Wirkstoffe

Diese können insbesondere sein Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Besonders bevorzugte Wirkstoffe sind Pflanzenextrakte wie beispielsweise Moringa Pteryosperma Seed Extract (wie beispielsweise Purisoft^{™} LS 9726 von BASF), Argania Spinosa Leaf Extract (wie beispielsweise Arganyl^{™} LS 9781 und ARGANYL PW LS 9830 von der BASF), Eperua Falcata Bark Extract (wie beispielsweise Eperuline^{™} PW von der BASF).

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z.B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt. Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen^{®} CAT, BASF Personal Care and Nutrition GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen Zubereitung - enthalten.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan^{®} von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl) phenol, 2-Benzyl-4-chlorphenol, 3-(4-Chlorphenoxy)-1,2-propandiol, 3-lod-2-propinylbutyl-carbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakterizide bzw. bakteriostatische Wirkstoffe in Mengen von 0,01 bis 5 und vorzugsweise 0,1 bis 2 Gew.-% - bezogen auf das Gesamtgewicht der kosmetischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierenden Substanzen in Mengen von 0,1 bis 30, vorzugsweise 1 bis 20 und insbesondere 2 bis 8 Gew.-% - bezogen auf das Gesamtgewicht der kosmetischen Zubereitung - enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide neben Vaseline und Paraffinen und Paraffinwachsen verwendet werden, wobei Fettsäurealkanolamide gleichzeitig als Schaumstabilisatoren dienen.

### UV-Filter

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis(7,7-dimethyl-2-oxobicyclo-[2.2.1]heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1 ,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1-hexyloxy)-1,3,5-triazin und 2,4,6-Tris[p-(2-ethylhexyl-oxycar-bonyl) anilino]-1 ,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb^{®} HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX);
➢ Tris-biphenyl Triazine (TinosorbA2B).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul^{®} A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung der EU-Richtlinie 2005/9/EC vom 28. Januar 2005 zur Änderung der Richtlinie 76/768/EEC, betreffend kosmetische Produkte, zum Zweck der Anpassung von Annex VII an den technischen Fortschritt) zugelassen Stoffe, auf die hier explizit Bezug genommen wird.

Neben den genannten löslichen Stoffen kommen für diesen Zweck auch unlösliche **Lichtschutzpigmente**, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T, Eusolex^{®} T-2000, Eusolex^{®} T-Aqua, Eusolex^{®} AVO, Eusolex^{®} T-ECO, Eusolex^{®} T-OLEO und Eusolex^{®} T-S (Merck). Typische Beispiele für sind Zinkoxide, wie z.B. Zinc Oxide neutral, Zinc Oxide NDM (Symrise) oder Z-Cote^{®} (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 sowie Parf.Kosm. 80. Jahrgang, Nr. 3/1999, S. 10 bis 16 zu entnehmen.

Neben den beiden vorgenannten Gruppen primärer Lichtschutzstoffe können auch **sekundäre Lichtschutzmittel** vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. - Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, - Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine bevorzugte Ausführungsform der Erfindung betrifft kosmetischen Zubereitungen, enthaltend eines oder mehrere erfindungsgemäße Polymere und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Tris-Biphenyl Triazine, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxa-nyl)propyl) phenol, Dimethicodiethylbenzalmalonate, unlösliche Lichtschutzpigmente und ihren Mischungen.

Diese UV-Lichtschutzfilter sind beispielsweise unter den folgenden Handelsnamen kommerziell erhältlich:
NeoHeliopan^{®}MBC (INCI: 4-Methylbenzylidene Camphor; Hersteller: Symrise); NeoHeliopan^{®} BB (INCI: Benzophenone-3, Hersteller: Symrise); Parsol^{®}1789 (INCI: Butyl Methoxydibenzoylmethane, Hersteller: Hoffmann-La Roche (Givaudan); Tinosorb^{®}S (INCI: Bis-Ethylhexyloxyphenol Methoxyphenyl Triazine); Tinosorb^{®}M (INCI: Methylene Bis-Benzotriazolyl Tetramethylbutylphenol): Herstelller: BASF; Uvasorb^{®}HEB (INCI: Diethylhexyl Butamido Triazone, Hersteller: 3V Inc.), Uvinul^{®}T 150 (INCI: Ethylhexyl Triazone, Hersteller: BASF AG); Uvinul^{®} A plus (INCI: Diethylamino Hydroxybenzoyl Hexyl Benzoate: Hersteller: BASF AG; Mexoryl^{®} SO: 3-(4'-Trimethylammonium) benzyliden- bornan-2-on-methylsulfat, INCI: Camphor Benzalkonium Methosulfate; Mexoryl^{®}SX: 3,3'-(1,4-Phenylendimethin)-bis (7,7-dimethyl-2-oxobicyclo-[2.2.1]heptan-1-methansulfonsäure), CTFA: INCI Terephthalylidene Dicamphor Sulfonic Acid; Mexory^{®} SL: 3-(4'-Sulfo)-benzyliden-bornan-2-on, INCI Benzylidene Camphor Sulfonic Acid; Mexoryl^{®}SW: Polymer von N-{(2und 4)-[2-oxoborn-3-yliden)methyl}benzyl]acrylamid, INCI Polyacrylamidomethyl Benzylidene Camphor; Mexoryl^{®}SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol^{®} SLX: Dimethicodiethylbenzalmalonate, INCI Polysilicone-15.

Weitere Beispiele sind **pigmentäre Lichtfilter**, wie beispielsweise anorganische pigmentäre Lichtfilter, wie Titandioxid und Zinkoxid und/oder organische pigmentäre Lichtfilter, wie Methylen-bis-benzotriazolyltetramethylbutylphenol (Tinosorb M) und Tris-Biphenyl Triazine (Tinosorb A2B).

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen Zubereitung - enthalten.

### Selbstbräuner

Als Selbstbräuner sind Substanzen zu verstehen, welche eine Bräunung der Haut verursachen. Beispielsweise seien Dihydroxyaceton, Erythrulose sowie alpha, betaungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose, Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o. g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die Selbstbräuner sind üblicherweise in Konzentrationen von 1 bis 10, insbesondere von 2 bis 5 Gew.-% -bezogen auf das Gesamtgewicht der kosmetischen Zubereitung, enthalten.

### Pigmente

Der Begriff Pigment umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als anorganische Pigmente seien exemplarisch genannt: Titandioxid, optional oberflächenbeschichtet, Zirkonium oder Ceriumoxide und Zink-, Eisen- (Schwarz, Gelb oder Rot) und Chromoxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus. Die anorganischen Pigmente können farbgebend sein. Besonders bevorzugte, anorganische, farbgebende Pigmente sind Metalloxide und insbesondere das Eisenoxid.

Die Pigmente können sowohl einzeln als auch in Mischungen vorliegen.

Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen können. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Folgende Pigmente können ebenfalls erfindungsgemäß verwendet werden.

Pigmente auf Glimmerbasis:
Cellini^{®}, Cloisonne^{®}, Duocrome^{®}, Flamenco^{®}, Gemtone^{®}, Timica^{®},
MultiReflections^{™}
Das Substrat Glimmer wird mit Metalloxiden - Titandioxid (TiO₂) und/oder Eisenoxid (Fe₂O₃), um weiße, interferierende und metallische Effekte zu erzeugen. Die reflektierende Farbe hängt von der Dicke der Metalloxidbeschichtung ab. Zur Erzegung von absorption und komplexer Farbwanderungseffekte, werden ebenfalls zusätzliche Pigmente verwendet. Diese anorganischen und organischen Pigmente sind Chromoxid, Eisenoxide, Eisenferrocyanid (Eisenblau), Carmin und D&C/FD&C-Farben.

Pigmente auf Basis Calciumnatriumborsilicat:
Plättchen aus Reflecks^{™}, Reflecks^{™} Dimensions, und Reflecks^{™} MultiDimensions oder anderen Farbstoffen werden mit Metalloxiden und/oder anderen Farbmitteln beschichtet, um Effektpigmente mit verstärkter Farbreinheit, Helligkeit, Transparenz und Reflexionsvermögen zu erzeugen. Reflecks^{™} MultiDimensions besteht aus Plättchen aus Calciumnatriumborsilicat, die mit Titandioxid und Siliciumdioxid (SiO₂) beschichtet sind. Diese Pigmente ermöglichen unverwechselbare Farbwanderungseffekte.

Pigmente auf Basis von synthetischem Glimmer:
Chione^{™}
Metalloxide sind auf Substrate aus synthetischem Glimmer aufgetragen. Auf grund der sauberen Hauptfarbe des Substrats ist das daraus entstandene Aussehen durch sehr helle Farben mit verstärkter Chroma charakterisiert. Diese Pigmente auf Basis von synthetischem Glimmer sind für die Verwendung in solchen Produkten ideal, wo breite Perlglanzeffekte und irisierende Effekte gewünscht sind.

Pigmente auf Basis von Wismuthoxichlorid:
Biju^{®}, Mearlite^{®}, Pearl-Glo^{®}, Chroma-Lite^{®}
Plättchen aus Wismuthoxichloridkristallen warden aus einer Lösung gefällt. Jeder Grad hat seine eigene Größe, Form, Dicke und Schimmer. Möglich sind auch Pigmente aus dem Bereich Chroma-Lite, wo Wismuthoxichlorid mit Glimmer und zusätzlichen Farbmitteln kombiniert sind.

Specialty Performance Minerals:
Mearlmica^{®}, Chione^{™} M-SVA, Bi-Lite^{®},
Specialty performance minerals sind eine Familie aus Mineralprodukten, die eine Balance und Konsistenz in gewisse Typen von kosmetischen Zusammensetzungen abgeben, während diese optisch neutral bleiben. Sie bewirken ebenfalls eine verbesserte Textur, insbesondere in Pulveranwendungen.

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Die erfindungsgemäßen Zubereitungen enthalten üblicherweise 0,1 bis 40 Gew.-% Pigmente - bezogen auf das Gesamtgewicht der kosmetischen Zubereitung.

Es ist ferner vorteilhaft im Sinne der vorliegenden Erfindung, wenn die erfindungsgemässe Zubereitung einen oder mehrere **Farbstoffe** enthält.

Die Farbstoffe können sowohl synthetischen als auch natürlichen Ursprungs sein. Eine Liste von geeigneten Farbstoffen findet sich in EP 1 371 359 A2, S.8, Z. 25-57, S.9 und S.10 sowie S.11, Z. 1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen Zubereitung.

Als Farbstoffe und Pigmente eignen sich insbesondere die gemäß Annex IV der Kommissions Direktive zugelassenen Farbstoffe und Pigmente (in der Fassung: EU-Richtlinie 2005/9/EC vom 28. Januar 2005 zur Änderung der Richtlinie 76/768/EEC, betreffend kosmetische Produkte, zum Zweck der Anpassung von Annex VII an den technischen Fortschritt) zugelassen Stoffe, auf die hiermit explizit Bezug genommen wird.

### Anorganische und organische Füllstoffe

### Puderrohstoffe / Füllstoffe / Sensorikmodifikatoren

Geeignete Füllstoffe können Talk, Siliciumoxid, Zinkstearat, Glimmer, Kaolin, Nylon (insbesondere Orgasol)pulver, Polyethylenpulver, Polypropylenpulver, Acrylatpulver, Teflon, Stärke, Bornitrid, Copolymermikrokugeln, wie Expancel (Nobel Industrie), Polytrap (Dow Coming) und Siliconharzmikroperlen (Tospearl from Toshiba).

Andere Füllstoffe, die in den Zusammensetzungen der Erfindung verwendet werden können, umfassen andere anorganische Pulver wie Kalk, hochdisperse Kieselsäure, Calciumoxid, Calciumcarbonat, Magnesiumoxid, Magnesiumcarbonat, Fuller's Erde, Attapulgit, Bentonit, Muscovit, Phlogopit, synthetischer Glimmer, Lepidolit, Hectorit, Biotit, Lithionglimmer, Vermiculit, Aluminumsilicat, Aluminummagnesiumsilicat, Diatomeenerde, Stärke, Alkyl- und/oder Trialkylarylammoniumsmectite, chemisch modifiziertes Magnesiumaluminumsilicat, organisch modifizierter Montmorillonitton, hydratisiertes Aluminumsilicat, hydratisierte Kieselsäure, hochdisperses Aluminumstärke-Octenyl succinatbariumsilicat, Calciumsilicat, Magnesiumsilicat, Strontiumsilicat, Metallwolframat, Magnesium, Siliciumoxid-Aluminiumoxid, Zeolit, Bariumsulfat, calciniertes Calciumsulfat (calcinierter Gips), Calciumphosphat, Fluorapatit, Hydroxyapatit, Keramikpulver, Metallseife (Zinkstearat, Magnesiumstearat, Zinkmyristat, Calciumpalmitat and Aluminumstearat), colloidales Siliciumdioxid; organisches Pulver, Cyclodextrin, Methylpolymethacrylatpulver, Copolymerpulver aus Styrol und Acrylsäure, Benzoguanaminharzpulver und Poly(ethylentetrafluorid) pulver; Siliciumoxiddimethylsilylat, Methylmethacrylat-Kreuzpolymer, Silsesquioxan, Vinyldimethicon/Methiconsilsesquioxan-Kreuzpolymer, Lauroyllysin.

### Emulgator

In einer Ausführungsform der Erfindung enthalten die erfindungsgemäßen Zubereitungen mindestens einen Emulgator.

Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus. Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Sucrose Ester (Saccharoseester), Alkylglucoside (z.B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydro-xystearate und Abmischungen davon, welche beispielsweise unter den Marken "Dehymuls^{®} PGPH" (W/O-Emulgator) oder "Eumulgin^{®}VL 75" (Abmischung mit Lauryl Glucosides im Gewichtsverhältnis 1:1, O/W-Emulgator) oder Dehymuls^{®} SBL (W/O-Emulgator) von der BASF Personal Care and Nutrition Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade^{®} Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/ Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als **lipophile W/O-Emulgatoren** eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitan-trimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der BASF Personal Care and Nutrition Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Weiterhin erfindungsgemäß vorteilhaft einsetzbar sind Sucrose Ester wie das Gemisch aus Sucrose Polystearate und Hydrogenated Polyisobutene, das unter der Bezeichnung EMULGADE^{®} Sucro im Handel ist. Sowie das Gemisch aus Sucrose Polystearate und Cetyl Palmitate, das unter der Bezeichnung EMULGADE^{®} Sucro Plus im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 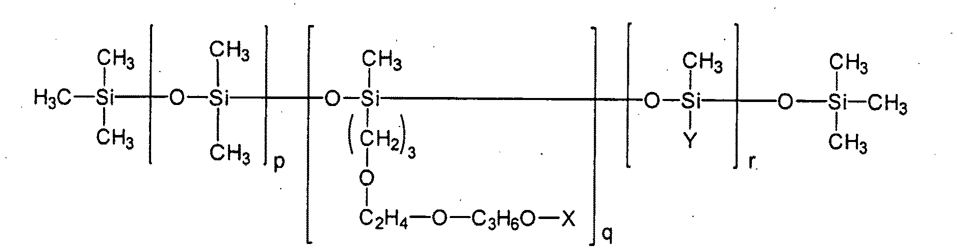 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Evonik unter den Warenbezeichnungen AXIL^{®} B 8842, ABIL^{®} B 8843, ABIL^{®} B 8847, ABIL^{®} B 8851, ABIL^{®} B 8852, ABILO B 8863, ABILO B 8873 und ABILOB 88183 verkauft werden. Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikEvonik unter der Warenbezeichnung ABIL^{®} EM 90 verkauft wird. Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Evonik unter der Warenbezeichnung ABIL^{®}EM 97 und ABIL^{®}WE 09 verkauft wird. Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning^{®} 5200 Formulation Aid von der Gesellschaft Dow Corning Ltd. erhältlich ist. Ein weiterer vorteilhafter Silikonemulgator ist Octyl Dimethicon Ethoxy Glucosid der Firma Wacker.

Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL^{®} EM 90 Evonik Evonik), DC5200 Formulation Aid (Dow Corning)] sowie beliebige Mischungen aus beiden Emulgatoren.

### Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächenaktive Substanzen verstanden, die einen HLB-Wert von größer 20 aufweisen.

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zubereitungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zubereitung.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfett¬säureester, Polyglycerinfettsäurester, Zuckerester, Sorbi¬tan¬ester, Poly¬sor¬bate und Aminoxide, Polyethermodifizierte Polysiloxane, Polyethermodifizierte Siloxanelastomere.. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾- oder - SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylme-thyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkali (z.B. Natrium und Kalium)-Salze, Ammonium- und Alkylammoniumsalze, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Alkyllactate, Alkyltartrate, Alkylcitrate, Alkylphosphate, N-Acylaminosäuren, wie beispielsweise Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Wachskomponente

In einer Ausführungsform der Erfindung enthalten die erfindungsgemäßen Zubereitungen mindestens eine Wachskomponente.

Die erfindungsgemäßen Zubereitungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett. Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der BASF Personal Care and Nutrition Deutschland GmbH & Co. KG vermarkteten Produkte Novata AB und Novata B (Gemisch aus C12-C18-Mono-, Di- und Triglyceriden), sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der BASF Personal Care and Nutrition Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder-stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Ölkörper

Das Öl und/oder Wachs in der erfindungsgemäßen texturierten Zusammensetzung ist aus der Gruppe Fettsäureester, Guerbetalkohole, Tri- oder Partialglyceride, Mono-/Dialkylether, Mono-/Dialkylcarbonate, öllösliche UV-Filter, Fettalkoholether, mikrokristalline Wachse, Kohlenwasserstoffe bzw. Mineralöl, Silikonöl, natürliche pflanzliche Öle und deren Mischungen ausgewählt.

Bevorzugte Öle und/oder Wachse sind Guerbetalkohole auf Basis von Fettalkoholen mit 6 bis 18, vorzugsweise 8 bis 10 Kohlenstoffatome (Eutanol G, Eutanol G 16) , Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat (Cetiol^{®} MM), Myristylpalmitat, Myristylstearat, Myristylisostearat, Myristyloleat, Myristylbehenat, Myristylerucat, Cetylmyristat, Cetylpalmitat (Cutina^{®} CP), Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Isopropyl Myristate, Isopropyl Palmitate, Oleylmyristat, Oleylpalmitat, Oleylstearat, Oleylisostearat, Oleyloleat, Oleylbehenat, Oleylerucat (Cetiol^{®} J 600), Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Ethylhexyl Stearate (Cetiol^{®} 868), Hexyl Laurate (Cetiol^{®} A), C12.15 Alkyl Benzoate (Cetiol^{®} AB), Dibutyl Adipate (Cetiol^{®} B), Coco-Caprylate (Cetiol^{®} C5), Coco-Caprylate/Caprate (Cetiol^{®} LC, Cetiol^{®} C 5C), Propylheptyl Caprylate (Cetiol^{®} Sensoft), Cetearyl Isononanoate (Cetiol^{®} SN), Decyl Oleate (Cetiol^{®} V), Cetearyl Ethylhexanoate (Luvitol^{®} EHO), Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) wie Propylene Glycol Dicaprylate/Dicaparte (Myritol^{®} PGDC). und/oder Guerbetalkoholen, Triglyceride auf Basis C₆-C₁₀-Fettsäuren flüssige Mono-/Di-/Triglyceridmischungen auf Basis von C₆-C₁₈-Fettsäuren (Myritol^{®} 331, Myritol^{®} 312, Myritol^{®} 318), , Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalko-holcarbonate, wie z. B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN, Cetiol^{®} AB), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen (Cetiol E). Weitere geeignete Emollients sind pflanzliche Öle (Cegesoft^{®} GPO, Cegesoft^{®} PFO, Cegesoft^{®} PS 6, Cegesoft^{®} SBE, Cegesoft^{®} SH) und Mischungen daraus (Cegesoft^{®} VP), Silikonöle, Kohlenwasserstoffe wie Mineralöle, Paraffinum Liquidum, Undecane / Tridecane (Cetiol^{®} Ultimate), Hydrogenated Polyisobutene (Luvitol^{®} Light), Mineralöle, Isoparaffine, Paraffine.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylylether (Cetiol^{®} OE), Ringöffnungsprodukte von epoxidierten Fettsäureestern mit Polyolen..

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 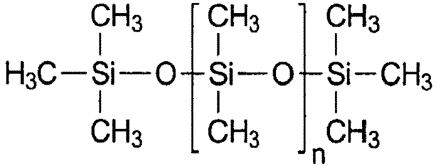 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Evonik erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Evonik erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan). Erfindungsgemäß besonders bevorzugte Silikone sind Dimethicon und Cyclomethicon.

Die erfindungsgemäßen Zubereitungen können weiterhin Konservierungsmittel, Parfümöle, Überfettungsmittel, Stabilisatoren und/oder Hydrotrope enthalten.

Als **Konservierungsmittel** eignen sich beispielsweise Phenoxyethanol, Formaldehydlösung, Parabene, Pentandiol oder Sorbinsäure, Benzoesäure und deren Salze, Benzyl Alcohol, Benzyl Salicylate. Dehydroacetic Acid, Methylthiazolinon oder Sorbinsäure und deren Salze sowie die unter der Bezeichnung Surfacine^{®} bekannten Silberkomplexe sowie die in Anlage 6, Teil A und B der Kosmetikverordnung aufgeführten weiteren Stoffklassen. Daneben kommen Substanzen zum Einsatz, die als Konservierungsmittelhelfer fungieren wie Ethylhexylglycerin und Caprylyl Glycol sowie Polyole bzw. Alcohol wie Propandiol, Phenylpropanol, Phenethyl Alkohol und Undecyl Alkohol, sowie die unter der Bezeichnung Surfacine^{®} bekannten Silberkomplexe. Weiterhin eignen sich als Konservierungsmittel die in WO 07/048757 beschriebenen 1,2 Alkandiole mit 5 bis 8 C-Atomen.

Als Konservierungsmittel eignen sich insbesondere die gemäß Annex VI der Kommissions Direktive (in der Fassung der EU-Richtlinie 2005/9/EC vom 28. Januar 2005 zur Änderung der Richtlinie 76/768/EEC, betreffend kosmetische Produkte, zum Zweck der Anpassung von Annex VII an den technischen Fortschritt) zugelassen Stoffe, auf die hier explizit Bezug genommen wird.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Stabilisatoren** können Metallsalze von Fettsäuren, wie z. B. Magnesium-, Aluminium- und/oder Zinkstearat bzw. -ricinoleat sowie Komplexbildner auch *Chelatbildner genannt.* Es sind chemische Verbindungen, die mit Metallionen Chelatkomplexe bilden. Sie führen zu einer Maskierung von (unerwünschten) chemischen Eigenschaften von Metallionen eingesetzt werden, wie z.B. Tetranatriumiminodisuccinat (auch: Iminodisuccinat-Tetranatriumsalz), Ethylendiamintetraessigsäure bzw. Ethylendiamintetraacetat.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### A1: Testverfahren zur Prüfung der Anti-Pollution-Wirkung

Um zu prüfen, ob Stoffe einen Anti-Pollution-Effekt für die menschliche Haut haben, insbesondere den Schutz der menschlichen Haut vor Staub, insbesondere vor Feinstaub bewirken, wurde das im Folgenden beschriebene Testverfahren herangezogen.

Als Schmutzmodell wurde Aktivkohle (erhalten von der Carl Roth GmbH, Karlsruhe, Deutschland) verwendet. Aktivkohle ist mikrokristalliner Kohlenstoff. Es besteht ein Größenunterschied zwischen den Partikeln von Aktivkohle und den Partikeln von Feinstaub. Trotzdem ist Aktivkohle eine geeignete Modellsubstanz für Feinstaub. Aktivkohle ist außerdem geeignet für Untersuchungen an menschlichen Probanden, da sie im Gegensatz zu vielen anderen Substanzen toxikologisch unbedenklich ist.

Für die Methode wurde ein Testaufbau entwickelt, um die antiadhäsiven Wirkung der zu prüfenden Stoffe auf der Haut untersuchen zu können. Die dabei verwendete Apparatur diente dem definierten Auftrag von Aktivkohle auf den Unterarmen der menschlichen Probanden. Dabei wurde eine definierte Menge Aktivkohle in eine Kammer gegeben und unter standardisiertem Druck über einen Luftstrom auf die Haut des Unterarms aufgetragen.

Die Testsubstanz wurde auf der mit Ethanol vorgereinigten Haut aufgetragen. Hierbei wurde eine standardisierte Menge der Testsubstanz auf ein definiertes Areal der Haut des Unterarms aufgetragen (wenn im Folgenden nichts anderes angegeben ist, waren dies 2 mg/cm²). Zunächst wurde vor der Behandlung jede Hautstelle mit einer Fotoaufnahme festgehalten. Nach dem Auftragen der Testsubstanz wurde eine weitere Fotographie der gleichen Stelle gemacht. Nach einer Trocknungszeit von 10 min wurde dann die Aktivkohle mittels Luftstoß aufgetragen. Nach der Behandlung mit Aktivkohle wurde ein weiteres Foto aufgenommen. Diese Aufnahmen diente dann der Ermittlung der anhaftenden Menge Schmutz (Aktivkohle) auf der Haut. Dieses Verfahren wurde für jede Testsubstanz an vier verschiedenen Stellen des Unterarms durchgeführt.

Nachdem die genannten Messungen an allen vier Stellen des Unterarms durchgeführt worden waren, wurden Unterschiede in der Abwaschbarkeit des anhaftenden Schmutzes (Aktivkohle) überprüft. Hierzu wurde eine definierte Menge Wasser über die jeweiligen Hautstellen gegeben.

Die gleichbleibenden Lichtverhältnisse wurden bei der Aufnahme der Fotos sichergestellt. Ein Vergleich der behandelten Areale wurde mittels einem geeigneten Computerprogramm durchgeführt. Hierbei wurde der mittlere Grauwert zugrunde gelegt. Der Wert der Haut mit anhaftender Aktivkohle wurde entsprechend von dem Wert derselben Hautstelle ohne Aktivkohle subtrahiert. Je höher der so erhaltene Differenzwert war, desto mehr Aktivkohle war an der Haut haften geblieben. Anschließend wurde der Mittelwert sowie die Standardabweichung von allen Probanden (wenn im Folgenden nichts anderes angegeben ist, waren dies 11) pro Probe berechnet. Die Abwaschbarkeit wurde bestimmt, indem der mittlere Grauwert nach dem Abwaschen von demjenigen vor dem Abwaschen subtrahiert wurde. Die Ergebnisse wurden relativ zu den Ergebnissen der unbehandelten Haut angegeben.

### A1-1: Testverfahren zur Prüfung der Anti-Pollution-Wirkung auf Haare

In einem ähnlichen Verfahren, wurde die Wirkung auf Haaren untersucht: Einzelhaare wurden mit einer Testsubstanz behandelt und anschließend einer definierten Menge Aktivkohle mit einer entsprechenden Apparatur über einen Luftstrom in einer Kammer ausgesetzt. Die Einzelhaare wurden mit einem Digitalmikroskop fotografiert und der Grad der Anschmutzung durch Bildauswertung quantifiziert. Die Einzelhaare wurden mit einer definierten Menge mit Wasser gespült und erneut digitalmikroskopisch fotografiert und die Anschmutzung entsprechend ermittelt.

### A2: Verfahren zur Bestimmung der Glasübergangstemperatur Tg

Die Bestimmung der Glasübergangstemperatur Tg erfolgte wie folgt. Polymere, die als wässrige Dispersion vorlagen, wurden vor der Messung gefriergetrocknet. Danach wurden sie, wie alle übrigen Polymere auch, in dem verwendeten DSC-Gerät (TA Instruments DSC Q100) einem Trocknungsverfahren unterzogen. Die Proben wurden einem isothermen Lauf bei 80°C für 1 Stunde unterworfen. Nach diesem Lauf wurden weitere 30-minütige isotherme Läufe durchgeführt, bis ein konstantes Gewicht der Probe erreicht wurde. Die Bestimmung der Polymer-Glastemperaturen Tg wurde mit demselben Instrument im MDSC-Modus (DSC-Messung) durchgeführt. Standard-Aluminiumpfannen, die 2 bis 10 mg des getrockneten Polymers enthielten, wurden im Temperaturbereich von -50° C bis 200° C unter Verwendung einer Aufheizrate von 1 K / min und einer Modulationsrate von 0,5 K/min erhitzt. Das reversible Heizsignal wurde nach dem Standard "Glass / Step Transition" der Software "Universal Analysis 2000" (Version 4.7A, TA Instruments, Eschborn / Deutschland) ausgewertet. Die Glastemperatur wurde als Wendepunkt der Kurve angegeben.

### B: Getestete Substanzen

Getestet wurden die im Folgenden beschriebenen Polymere 1 bis 9. % bedeuten Gew.-%.

Das Polymer 1 ist nicht erfindungsgemäß.

### Erste Gruppe: Polymere oder Copolymere der Acrylsäure oder der Methacrylsäure

| **Polymer Nr.** | **Bezeichnung** | **Genauere Bezeichnung** |
|---|---|---|
| Polymer 1 | Carbomer (INCI-Bezeichnung für Polyacrylsäure) | Acrylsäure, vernetzt (mit weniger als 1 Gew.-% Vernetzer) |
| Polymer 2 | ein Acrylat-Copolymer | Ein Copolymer zusammengesetzt aus Ethylacrylat (30-60%), Methacrylsäure (30-60%), Methacrylamid (5-30%), C18-Alkyl-(EO)25-Methacrylat (0.1-10%), Pentaerythrittriallylether (0,01-1,0%) |
| Polymer 3 | ein Acrylat-Copolymer | Ein Copolymer aus Ethylacrylat (30-60%) und Methacrylsäure (40-70%) |
| Polymer 3a | ein Acrylat-Copolymer | Ein Copolymer aus Ethylacrylat (40-60%, Methacrylsäure (30-50%), Acrylsäure (5-15%), Pentaerythrittriallylether (0,01-1,0%) |
| Polymer 4 | ein Acrylat-Copolymer | Ein Terpolymer aus tert-Butylacrylat (60-80%), Ethylacrylat (5-20%) und Methacrylsäure (10-30%) |

Anmerkung zur Bezeichnung: Unter "Acrylat" wird verstanden Acrylsäure und Methacrylsäure sowie deren Salze und Ester

### Zweite Gruppe: kationische Polymere

| **Polymer Nr.** | **Bezeichnung** | **Genauere Bezeichnung** |
|---|---|---|
| Polymer 5 | Polyquaternium-68 (INCI) | Ein Copolymer aus Vinylpyrrolidon (40-65%), Methacrylamid (25-35%), Vinylimidazol (5-20%) und quaternärem (methyliertem) Vinylimidazol (5-20%) |

### Dritte Gruppe: Caprolactam-haltige Polymere

| **Polymer Nr.** | **Bezeichnung** | **Genauere Bezeichnung** |
|---|---|---|
| Polymer 6 | Polyvinylcaprolactam | N-Vinylcaprolactam Homopolymer |

### Vierte Gruppe: Copolymere aus Vinylpyrrolidon (VP), Vinylimidazol (VI) und fakultativ weiteren Monomeren

| **Polymer Nr.** | **Bezeichnung** | **Genauere Bezeichnung** |
|---|---|---|
| Polymer 7 | VP/Methacrylamid/VI Copolymer | Ein Copolymer aus Vinylpyrrolidon (50-70%), Methacrylamid (20-40%) und Vinylimidazol (1-10%) |

### Vergleichsgruppe: Polycarbonate und Polyurethane

| **Polymer Nr.** | **Bezeichnung** | **Genauere Bezeichnung** |
|---|---|---|
| Polymer 8 | Hydriertes Dimer Dilinoleyl / Dimethylcarbonat Copolymer | Hydrogenated Dimer Dilinoleyl / Dimethylcarbonate Copolymer |
| Polymer 9 | Polyurethan-39 (INCI) | Polyurethan |

### C: Eigenschaften der getesteten Polymere und Test-Ergebnisse

(Anti-Adhäsion/Abwaschbarkeit gemessen mit Proben mit 1 Gew.-% Polymer in Wasser):

| **Polymer Nummer** | **Löslichkeit mindestens 0.1g/100 g Wasser** | **Mittlere molare Masse mindestens 100000 g/mol (Gewichtsmittel Mw)** | **Glasübergangstemperatur über 70 °C** | **Anti-adhäsiver Effekt im Vergleich zur unbehandelten Haut (mindestens 5% geringere Adhäsion)** | **Weniger partikuläre Substanzen auf der Haut nach Abwaschen (mindestens 5%) im Vergleich zur unbehandelten Haut** |
|---|---|---|---|---|---|
| 1 | ja | ja | ja | ja | ja |
| 2 | ja | ja | ja | ja | ja |
| 3 | ja | ja | ja | ja | ja |
| 3a | ja | ja | ja | ja | ja |
| 4 | ja | ja | ja | ja | ja |
| 5 | ja | ja | ja | ja | ja |
| 6 | ja | ja | ja | ja | ja |
| 7 | ja | ja | ja | ja | ja |
| 8 | nein | nein | nein | nein | nein |
| 9 | ja | nein | nein | nein | nein |

Diese Ergebnisse zeigen, dass die Polymere 1 bis 7 den gewünschten Anti-Pollution-Effekt hervorbringen, die Polymere 8 und 9 jedoch nicht. Es kann davon ausgegangen werden, dass diejenigen Polymere, die Schutz gegen Staub bewirken, auch einen weitergehenden und allgemeineren Schutz gegen Luftverschmutzung bewirken, nicht nur gegen Staub, sondern auch gegen andere Stoffe, die die Luftverschmutzung ausmachen.

### C-1: Test-Ergebnisse auf Haaren

| **Polymer Nummer** | **Anti-adhäsiver Effekt im Vergleich zur unbehandelten Haar (mindestens 5% geringere Adhäsion im Vergleich zum unbehandelten Haar)** | **Weniger partikuläre Substanzen auf dem Haar nach Abgespült (mindestens 5%) im Vergleich zum unbehandelten Haar** |
|---|---|---|
| 2 | nein | ja |
| 3 | ja | ja |
| 5 | ja | ja |
| 9 | nein | nein |

Diese Ergebnisse zeigen, dass das auch bei Haaren die Polymere 3 und 5 den gewünschten Anti-Pollution-Effekt hervorbringen, das Polymere 9 jedoch nicht.

### D: Rezepturen

Die folgenden Rezepturen zeigen beispielhaft, wie kosmetische Produkte zusammengesetzt sein können, die die oben genannten Polymere enthalten und also einen Anti-Pollution-Effekt hervorbringen sollten.

Die Zahlenangaben bezeichnen Gewichts-%.

Zum Teil werde die Polymere bereits als Lösung oder in anderer nicht reiner Form in die Formulierung eingebracht. In diesen Fällen ist zusätzlich der Aktivgehalt an Polymer angegeben.

Die Rezeptur 1 ist nicht erfindungsgemäß.

### Rezeptur 1: halbfestes Gel (Creme), Rezeptur 2-7: dünnflüssige bis fließfähige Gel-Lotions

| **Bestandteile** | **Chemische Bezeichnung / Zusammensetzung (%)** | **1** | **2** | **3** | **4** | **5** | **6** | **7** |
|---|---|---|---|---|---|---|---|---|
| Polymer 1 | | 1,00 | | | | | | |
| Polymer 2 | | | 3,30 (= 1.0 Aktivgehalt) | | | | | |
| Polymer 3 | | | | 3,30 (= 1.0 Aktivgehalt) | | | | |
| Polymer 4 | | | | | 1,00 | | | |
| Polymer 5 | | | | | | 5.,00 (= 1.0 Aktivgehalt) | | |
| Polymer 6 | | | | | | | 2,50 (= 1.0 Aktivgehalt) | |
| Polymer 7 | | | | | | | | 5,00 (= 1.0 Aktivgehalt) |
| Wasser, demin. | Wasser | Add 100 | Add 100 | Add 100 | Add 100 | Add 100 | Add 100 | Add 100 |
| Cetiol^{®} Ultimate | Undecan (und) Tridecan | 2.,50 | | 1,00 | | | 0,50 | |
| Cyclomethicone | Cyclomethicon | | 1,00 | | 1,00 | | 0,50 | |
| Tocopherol | Tocopherol (Vitamin E) | 0,10 | | | | 0,20 | | 0,50 |
| Cegesoft^{®} PS 6 | Olus Oil | | | 0,2 | | | 0,1 | |
| Tinosorb^{®} M | Methylen Bis-Benzotriazolyl Tetramethylbutylphenol (und) Aqua (und) Decyl Glucosid (und) Propylen Glycol (und) Xanthan Gum | 5,00 | | | | 10,00 | | |
| Z-Cote^{®} HP1 | Zinc Oxide (nano), Triethoxycaprylylsilane | 1,00 | | 1,00 | | | 4,00 | |
| T-80AS | Titanium Dioxid, Silica, Triethoxy Caprylylsilan | | 10,00 | 5,00 | 10,00 | | 2,00 | |
| D-Panthenol USP | Panthenol | | | 0,5% | | | | |
| Glycerin | Glycerin | 5.,00 | 1,00 | 5,00 | 1,00 | 0,5 | 1,00 | |
| PatcH2O^{®} | Water (und) Glycerin (und) Trehalose (and) Urea (und) Serine (und) Pentylen Glycol (und) Glyceryl Polyacrylat (und) Algin (and) Caprylyl Glycol (und) Natrium Hyaluronat (und) Pullulan (und) Dinatrium Phosphat (und) Kalium Phosphat | 3,00 | | | | 1,5 | | 3,00 |
| Hydagen^{®} GG | Glyceryl Glucosid und Glycerin | | | | | | 5,00 | 12,00 |
| PURISOFT POE LS 9726 | Water (und) Glycerin (und) Moringa Pteryosperma Seed Extract | | 3,00 | | | 3,00 | | 3,00 |
| Talc FM SSA *(K.S. Pearl)* | Talc (and) Dimethicon | 5,00 | | | | 1,00 | | |
| Mearlmica^{®} Treated SVA | Mica, Lauroyl Lysin | | | 5,00 | | | | 1,00 |
| Pearl-Glo^{®} SF PG1099 | Bismuth Oxychlorid | | 5,00 | | 5,00 | | | |
| SA-SB-300 *(Miyoshi Kasei)* | Silica | | | | | 2,00 | | 1,00 |
| *Dow Corning* 9701 Cosmetic Powder (Dow Corning) | Dimethicon/Vinyl Dimethicon Crosspolymer (and) Silica | | | | | 2,00 | | 1,00 |
| STR-100A-LP *(Sakai Chemical Industry)* | Titanium Dioxid (nano), Hydrated Silica, Dimethicon /Methicon Copolymer, Aluminum Hydroxid | | | | | | 5,00 | |
| Timica^{®} Terra White MN4501 | Mica, Titanium Dioxid | 1,00 | 1,00 | 0,5 | 1,00 | 0,5 | 0,5 | 1,00 |
| Timica^{®} Terra Brown MN4509 | Mica, Eisenoxide Titanium Dioxid | 0,1 | | 0,1 | | | | |
| Timica^{®} Terra Yellow MN4502 | Mica, Eisenoxide, Titanium Dioxide | | 0,1 | 0,1 | 0,1 | | 0,1 | |
| Flamenco^{®} Silk Blue 630M | Mica, Titanium Dioxide | | | | | | 0,5 | 1,00 |
| KOH 20%ig | Kaliumyhydroxid | | zur Anpassung an pH 6.5-7.0 | | | | | |
| Euxyl^{®} K 320 | 2-Phenoxyethanol, Methyl-4-hydroxybenzoate, Ethyl-4-hydro-xybenzoate | | 1.,00 | | 1,00 | 1,30 | 1,30 | 1,00 |
| Protectol^{®} PE | Phenoxyethanol | 1,00 | | 1,00 | | | | |
| Sensiva^{®} SC 50 | Ethylhexylglycerin | 0,5 | | 0,50 | | | | |
| **PH Wert** | | | **6,8** | **6,6** | **6,5** | **6,4** | **6,5** | **6,6** |

### Rezepturen 8-13: Gel-Lotions applizierbar als Pumpspray und Aerosolspray als Hautschutzmittel und / oder als Top-Coat und / oder Base-Coat zur additiven Anwendung zu Hautpflegezubereitungen

| **Bestandteile** | **Chemische Bezeichnung / Zusammensetzung (%)** | **8** | **9** | **10** | **11** | **12** | **13** |
|---|---|---|---|---|---|---|---|
| Polymer 3 | | 3,30 (= 1.0 Aktivgehalt) | 1,65 (= 0.5 Aktivgehalt) | 2,20 | 0,33 (= 0.1 Aktivgehalt) | 1,65 (= 0.5 Aktivgehalt) | 0,33 (= 0.1 Aktivgehalt) |
| Tinovis^{®} GTC UP | Hydrophob modifizierte Copolymere der Acrylsäure Acrylat/Beheneth-25 Methacrylat Copolymer ∼30%ige wässrige Lösung | | | 0,33 (= 0.1 Aktivgehalt) | | 0,33 (= 0.1 Aktivgehalt) | 0,33 (= 0.1 Aktivgehalt) |
| Wasser, demin. | Wasser | Add 100 | Add 100 | Add 100 | Add 100 | Add 100 | Add 100 |
| Cetiol^{®} Ultimate | Undecan (und) Tridecan | 1,00 | 1,00 | | | 0,50 | |
| | | | | | | | |
| Cyclomethic one | Cyclomethicon | | | | | 0,50 | |
| Tocopherol | Tocopherol (Vitamin E) | 0,10 | | | 0,20 | | 0,50 |
| Cegesoft^{®} PS 6 | Olus Oil | | 0.,2 | | | 0,1 | |
| Tinosorb^{®} M | Methylen Bis-Benzotriazolyl Tetramethylbutylphenol (und) Aqua (und) Decyl Glucosid (und) Propylen Glycol (und) Xanthan Gum | 1,00 | | | 1.,00 | | |
| Z-Cote^{®} HP1 | Zinc Oxide (nano), Triethoxycaprylylsil an | | 1,00 | | | 1,00 | |
| T-80AS | Titanium Dioxid, Silica, Triethoxy Caprylylsilan | | | 1,00 | | | |
| D-Panthenol USP | Panthenol | | 0,5 | | | | 0,5 |
| Glycerin | Glycerin | 3,00 | 5,00 | 1,00 | 2.,0 | 1,00 | |
| PatcH2O^{®} | Wasser (und) Glycerin (und) Trehalose (and) Urea (und) Serine (und) Pentylen Glycol (und) Glyceryl Polyacrylat (und) Algin (and) Caprylyl Glycol (und) Natrium Hyaluronat (und) Pullulan (und) Dinatrium Phosphat (und) Kalium Phosphat | | | | 1,5 | | 3,00 |
| Hydagen^{®} GG | Glyceryl Glucosid und Glycerin | | | | | 5,00 | 12,00 |
| PURISOFT POE LS 9726 | Wasser (und) Glycerin (und) Moringa Pteryosperma Seed Extract | | 3,00 | | 1,00 | | 1,50 |
| Talc FM SSA *(K.S.Pearl)* | Talc (and) Dimethicon | 1,00 | | | 1,00 | | |
| Mearlmica^{®} Treated SVA | Mica, Lauroyl Lysine | | 2,00 | | | | 1,00 |
| Pearl-Glo^{®} SF PG1099 | Bismuth Oxychloride | | | 1,00 | | | |
| SA-SB-300 *(Miyoshi Kasei)* | Silica | | | | 0,5 | | |
| *Dow Corning* 9701 Cosmetic Powder (Dow Corning) | Dimethicone/Vinyl Dimethicone Crosspolymer (and) Silica | | | | | 2,00 | 1,00 |
| STR-100A-LP *(Sakai Chemical Industry)* | Titanium Dioxide (nano), Hydrated Silica, Dimethicone /Methicone Copolymer, Aluminum Hydroxide | | | | | 5,00 | |
| Timica^{®} Terra White MN4501 | Mica, Titanium Dioxide | 1,00 | 0,5 | 1,00 | 0,5 | 0,5 | |
| Timica^{®} Terra Brown MN4509 | Mica, Iron Oxides, Titanium Dioxide | 0,1 | 0,1 | | | | |
| Timica^{®} Terra Yellow MN4502 | Mica, Iron Oxides, Titanium Dioxide | | 0,1 | 0,1 | | 0,1 | |
| Flamenco^{®} Silk Blue 630M | Mica, Titanium Dioxide | | | | | 0,5 | 1,00 |
| KOH 20%ig | Kalium Hydroxid | Zur Anpassung an pH 6.5-7.0 | | | | | |
| Euxyl^{®} K 320 | 2-Phenoxyethanol, Methyl-4-hydroxybenzoate, Ethyl-4-hydroxybenzoate | | | 1,00 | 1,30 | 1.,30 | 1,00 |
| Protectol^{®} PE | Phenoxyethanol | 1,00 | 1,00 | | | | |
| Sensiva^{®} SC 50 | Ethylhexylglycerin | 0,50 | 0,50 | | | | |
| PH Wert | | 6,8 | 6,6 | 6,5 | 6,4 | 6,5 | 6,6 |

Die Rezepturen 14-19 sind nicht erfindungsgemäß.

### Rezepturen 14-19: Cremes als Hautschutzmittel zur Tagespflege, Babypflege, Gesichtspflege, Körperpflege

| **Bestandteile** | **Chemische Bezeichnung / Zusammensetzung (%)** | **14** | **15** | **16** | **17** | **18** | **19** |
|---|---|---|---|---|---|---|---|
| Polymer 1 | | 0,2 | 0,2 | 0,1 | 0,5 | 0,1 | 0,5 |
| Tinovis^{®} GTC UP | Hydrophob modifizierte Copolymere der Acrylsäure Acrylat/Beheneth-25 Methacrylat Copolymer -30%ige wässrige Lösung | | 3,33 (= 1 Aktivgehalt) | 1,65 (= 0.1 Aktivgehalt) | 1,65 (= 0.5 Aktivgehalt) | 0,33 (= 0.1 Aktivgehalt) | 0,33 (= 0.1 Aktiv gehal t) |
| Wasser, demin. | Wasser | Add 100 | Add 100 | Add 100 | Add 100 | Add 100 | Add 100 |
| Emulgade PL 68/50 | Cetearyl Glucosid und Cetearyl Alcohol | 1,00 | | 1,00 | | | |
| Emulgade Sucro Plus | Sucrose Polystearate und Cetyl Palmitat | | 1,50 | | | | |
| Eumulgin VL 75 | Lauryl Glucosid (und) Polyglyceryl-2 Dipolyhydroxystearat (und) Glycerin | | | | 1,50 | | |
| Eumulgin Prisma | Disodium Cetearyl Sulfosuccinate | | | | | 0,2 | |
| AXOL^{®} C 62, Evonik | Glyceryl Stearate Citrat | | | | | | 1,00 |
| Cetiol^{®} Ultimate | Undecan (und) Tridecan | 2,50 | 2,50 | 1,25 | 5,00 | 0,50 | 2,50 |
| Cyclomethic one | Cyclomethicon | | | | | 2,50 | 0,50 |
| Tocopherol | Tocopherol (Vitamin E) | 0,10 | | | 0,20 | | 0,50 |
| Cegesoft^{®} PS 6 | Olus Oil | | 0,20 | | | 0,1 | |
| Tinosorb^{®} M | Methylen Bis-Benzotriazolyl Tetramethylbutylphenol (und) Aqua (und) Decyl Glucosid (und) Propylen Glycol (und) Xanthan Gum | 1,00 | | | 5,00 | | |
| Z-Cote^{®} HP1 | Zinc Oxide (nano), Triethoxycaprylylsilan | | 1,00 | | | 1,00 | |
| T-80AS | Titanium Dioxid, Silica, Triethoxy Caprylylsilan | | | 1,00 | | | |
| D-Panthenol USP | Panthenol | | 0,50 | | | | 0,50 |
| Glycerin | Glycerin | 3,00 | 5,00 | 1,00 | 2,00 | 1,00 | |
| PatcH2O^{®} | Wasser (und) Glycerin (und) Trehalose (and) Urea (und) Serine (und) Pentylen Glycol (und) Glyceryl Polyacrylat (und) Algin (and) Caprylyl Glycol (und) Natrium Hyaluronat (und) Pullulan (und) Dinatrium Phosphat (und) Kalium Phosphat | | | | 1,50 | | 3,00 |
| Hydagen^{®} GG | Glyceryl Glucosid und Glycerin | | | | | 5,00 | 5,00 |
| PURISOFT POE LS 9726 | Wasser (und) Glycerin (und) Moringa Pteryosperma Seed Extract | | 3,00 | | 1,00 | | 1,50 |
| Talc FM SSA *(K.S. Pearl)* | Talc (and) Dimethicon | 1,00 | | | 1,00 | | |
| Mearlmica^{®} Treated SVA | Mica, Lauroyl Lysine | | 2,00 | | | | 1,00 |
| Pearl-Glo^{®} SF PG1099 | Bismuth Oxychloride | | | 1,00 | | | |
| KOH 20%ig | Kalium Hydroxid | Zur Anpassung an pH 6.5-7.0 | | | | | |
| Euxyl^{®} K 320 | 2-Phenoxyethanol, Methyl-4-hydroxybenzoate, Ethyl-4-hydroxybenzoate | | | 1,30 | 1,30 | 1,00 | 1,00 |
| Protectol^{®} PE | Phenoxyethanol | 1,00 | 1,00 | | | | |
| Sensiva^{®} SC 50 | Ethylhexylglycerin | 0,50 | 0,50 | | | | |
| PH Wert | 6,8 | 6,6 | 6,5 | 6,4 | 6,5 | 6,6 | |

## Patentansprüche

1. Die kosmetische, nicht-therapeutische
Verwendung eines Polymers oder eines kosmetischen Produkts enthaltend dieses Polymer und zusätzlich andere, bekannte kosmetischen Inhaltsstoffe zur Herbeiführung eines Anti-Pollution-Effekts für menschliche Haut oder für menschliche Haare, insbesondere zum Schutz menschlicher Haut oder menschlicher Haare vor Staub, insbesondere vor Feinstaub,
wobei das Polymer eine Wasserlöslichkeit von mindestens 0,01 g Polymer in 100 g Wasser bei 20° C bei mindestens einem pH-Wert im Bereich zwischen 4 und 9 hat, und wobei das Polymer ausgewählt wird aus der Gruppe bestehend aus
• einem Copolymer aus Methacrylsäure und Ethylacrylat, welches optional außerdem Widerholungseinheiten enthält, die abgeleitet sind von mindestens einem Monomer ausgewählt aus der Gruppe bestehend aus Acrylsäure, Methacrylamid, einem Alkylacrylat mit 2 bis 30 C-Atomen in der Alkylgruppe, einem Alkylmethacrylat mit 2 bis 30 C-Atomen in der Alkylgruppe, einem Ester der Methacrylsäure mit einem C12- bis C30 Fettalkohol, wobei der Fettalkohol mit 1 bis 100 Ethylenoxideinheiten ethoxyliert ist, und einem Monomer, welches mindestens zwei polymerisierbare, ethylenisch ungesättigte Doppelbindungen enthält, so dass diese eine Verzweigung oder Vernetzung bewirken können, und
• einem Copolymer des Vinylpyrrolidon mit mindestens einem weiteren ethylenisch ungesättigten, polymerisierbaren Monomer, wobei der Anteil des Vinylpyrrolidon in diesem Copolymer höchstens 90 Gew.-%, bevorzugt höchstens 80 Gew.-%, insbesondere höchstens 70 Gew.-% beträgt,
wobei für den Fall, dass das Polymer Wiederholungseinheiten abgeleitet von Acrylsäure oder Methacrylsäure enthält, diese Wiederholungseinheiten vorliegen als freie Säuren oder als Salze, wobei, wenn sie als Salze vorliegen, die Salze bevorzugt Alkalisalze, insbesondere Natriumsalze sind.

2. Die kosmetische, nicht-therapeutische Verwendung nach Anspruch 1, wobei das Monomer, welches mindestens zwei polymerisierbare, ethylenisch ungesättigte Doppelbindungen enthält, Pentaerythrittriallylether ist.

3. Die kosmetische, nicht-therapeutische Verwendung nach Anspruch 1, wobei das Polymer ein Copolymer des Vinylpyrrolidon mit mindestens einem weiteren ethylenisch ungesättigten, polymerisierbaren Monomer ist, wobei der Anteil des Vinylpyrrolidon in diesem Copolymer höchstens 90 Gew.-%, bevorzugt höchstens 80 Gew.-%, insbesondere höchstens 70 Gew.-% beträgt, und wobei das mindestens eine weiteren ethylenisch ungesättigten, polymerisierbare Monomer ausgewählt wird aus der Gruppe bestehend aus Vinylimidazol, Methyl-Vinylimidazolium, zusammen mit einem beliebigen Anion, Acrylamid und Methacrylamid.

4. Die kosmetische, nicht-therapeutische Verwendung nach Anspruch 3, wobei das Anion ausgewählt wird aus der Gruppe bestehend aus Chlorid und (H₃C-O-SO₃).

5. Die kosmetische, nicht-therapeutische Verwendung nach einem der Ansprüche 1 bis 4, wobei das Polymer eine Glasübergangstemperatur von mindestens 70°C hat.

6. Die kosmetische, nicht-therapeutische Verwendung nach einem der Ansprüche 1 bis 5, wobei das Polymer eine mittlere molare Masse (Gewichtsmittel Mw) von mindestens 100000 g/mol hat.

7. Die kosmetische, nicht-therapeutische Verwendung nach einem der Ansprüche 1 bis 6, wobei das Polymer ausgewählt wird aus der Gruppe bestehend aus
• einem Copolymer zusammengesetzt aus 30-60 Gew.-% Ethylacrylat, 30-60 Gew.-% Methacrylsäure, 5-30 Gew.-% Methacrylamid, 0,1-10 Gew.-% C18-Alkyl-(EO)25-Methacrylate und 0,01-1,0 Gew.-% Pentaerythrittriallylether,
• einem Copolymer zusammengesetzt aus 30-60 Gew.-% Ethylacrylat und 40-70 Gew.-% Methacrylsäure,
• einem Copolymer zusammengesetzt aus 40-60 Gew.-% Ethylacrylat, 30-50 Gew.-% Methacrylsäure, 5-15 Gew.-% Acrylsäure, und 0,01-1,0 Gew.-% Pentaerythrittriallylether,
• einem Terpolymer aus 60-80 Gew.-% tert-Butylacrylat, 5-20 Gew.-% Ethylacrylat und 10-30 Gew.-% Methacrylsäure,
• einem Copolymer aus 40-65 Gew.-% Vinylpyrrolidon, 25-35 Gew.-% Methacrylamid, 5-20 Gew.-% Vinylimidazol und 5-20 Gew.-% quaternärem (methyliertem) Vinylimidazol, und
• einem Copolymer aus 50-70 Gew.-% Vinylpyrrolidon, 20-40 Gew.-% Methacrylamid und 1-10 Gew.-% Vinylimidazol.

8. Die kosmetische, nicht-therapeutische Verwendung nach einem der Ansprüche 1 bis 7, wobei ein kosmetisches Produkt enthaltend das Polymer und zusätzlich andere, bekannte kosmetischen Inhaltsstoffe verwendet wird, und wobei das Polymer in dem Produkt in einer Konzentration von 0,01 bis 5 Gew.-%, bevorzugt 0,05 bis 2 Gew.-%, bevorzugt 0,1 bis 1,5 Gew.-% enthalten ist.

9. Die kosmetische, nicht-therapeutische Verwendung nach Anspruch 8, wobei das kosmetische Produkt neben dem Polymer mindestens Wasser als zusätzlichen anderen, bekannten kosmetischen Inhaltsstoff enthält, und wobei das Produkt mindestens 20 Gew.-%, bevorzugt mindestens 50 Gew.-%, bevorzugt mindestens 99,9 Gew.-%, Wasser enthält, und wobei das Polymer bei 20°C vollständig in Wasser dispergiert, suspendiert, gelöst oder gequollen vorliegt.

10. Die kosmetische, nicht-therapeutische Verwendung nach einem der Ansprüche 1 bis 9, wobei ein kosmetisches Produkt enthaltend das Polymer und zusätzlich andere, bekannte kosmetischen Inhaltsstoffe verwendet wird, und wobei das kosmetische Produkt ein solches ist, das zum Schutz, zur Pflege und / oder zur Reinigung der Haut dient, oder ein solches ist, das zur Pflege und / oder zur Reinigung der Haare dient.

11. Die kosmetische, nicht-therapeutische Verwendung nach einem der Ansprüche 1 bis 9, wobei ein kosmetisches Produkt enthaltend das Polymer und zusätzlich andere, bekannte kosmetischen Inhaltsstoffe verwendet wird, und wobei das kosmetische Produkt ausgewählt ist aus der Gruppe bestehend aus einer wässrigen Polymerlösungen (d. h. einem Hydrogel), einer emulgatorfreien Emulsion (d. h. einer Gelcreme), einer Emulsion wie z. B. einer Creme oder einer Lotion.

12. Ein kosmetisches, nicht-therapeutisches Verfahren zur Herbeiführung eines Anti-Pollution-Effekts für menschliche Haut oder für menschliche Haare, insbesondere zum Schutz menschlicher Haut oder menschlicher Haare vor Staub, insbesondere vor Feinstaub, umfassend das Aufbringen des Polymers wie definiert in einem der vorangehenden Patentansprüche, oder umfassend das Aufbringen eines kosmetischen Produkts enthaltend das Polymers wie definiert in einem der vorangehenden Patentansprüche und zusätzlich enthaltend andere, bekannte kosmetischen Inhaltsstoffe, auf solche menschliche Haut oder solche menschlichen Haare, die eines Anti-Pollution-Effekts, insbesondere des Schutzes vor Staub, insbesondere des Schutzes vor Feinstaub, bedürfen.

## Claims

1. The cosmetic, non-therapeutic use of a polymer or a cosmetic product comprising said polymer and in addition other known cosmetic ingredients for invoking an anti-pollution effect on human skin or on human hair, particularly to protect human skin or human hair from particulate matter, especially fine particulates,
wherein the polymer has a water solubility of at least 0.01 g of polymer in 100 g of water at 20°C at at least a pH in the range between 4 and 9, and wherein the polymer is selected from the group consisting of
• a copolymer of methacrylic acid and ethyl acrylate which optionally also comprises repeat units derived from at least one monomer selected from the group consisting of acrylic acid, methacrylamide, an alkyl acrylate having 2 to 30 carbon atoms in the alkyl group, an alkyl methacrylate having 2 to 30 atoms in the alkyl group, an ester of methacrylic acid with a C12 to C30 fatty alcohol, wherein the fatty alcohol is ethoxylated with 1 to 100 ethylene oxide units, and a monomer which comprises at least two polymerizable ethylenically unsaturated double bonds such that said double bonds may effect branching or crosslinking, and
• a copolymer of vinylpyrrolidone with at least one further ethylenically unsaturated polymerizable monomer, wherein the proportion of vinylpyrrolidone in said copolymer is at most 90% by weight, preferably at most 80% by weight, especially at most 70% by weight,
wherein in the case that the polymer comprises repeat units derived from acrylic acid or methacrylic acid, said repeat units are present as free acids or as salts, wherein, if they are present as salts, the salts are preferably alkali metal salts, especially sodium salts.

2. The cosmetic, non-therapeutic use as claimed in claim 1, wherein the monomer which comprises at least two polymerizable ethylenically unsaturated double bonds is pentaerythritol triallyl ether.

3. The cosmetic, non-therapeutic use as claimed in claim 1, wherein the polymer is a copolymer of vinylpyrrolidone with at least one further ethylenically unsaturated, polymerizable monomer, wherein the proportion of vinylpyrrolidone in said copolymer is at most 90% by weight, preferably at most 80% by weight, especially at most 70% by weight, and wherein the at least one further ethylenically unsaturated, polymerizable monomer is selected from the group consisting of vinylimidazole, methylvinylimidazolium together with any anion, acrylamide and methacrylamide.

4. The cosmetic, non-therapeutic use as claimed in claim 3, wherein the anion is selected from the group consisting of chloride and (H₃C-O-SO₃) .

5. The cosmetic, non-therapeutic use as claimed in any of claims 1 to 4, wherein the polymer has a glass transition temperature of at least 70°C.

6. The cosmetic, non-therapeutic use as claimed in any of claims 1 to 5, wherein the polymer has an average molar mass (weight average Mw) of at least 100 000 g/mol.

7. The cosmetic, non-therapeutic use as claimed in any of claims 1 to 6, wherein the polymer is selected from the group consisting of
• a copolymer composed of 30-60% by weight ethyl acrylate, 30-60% by weight methacrylic acid, 5-30% by weight methacrylamide, 0.1-10% by weight C18-alkyl-(EO)25 methacrylates and 0.01-1.0% by weight pentaerythritol triallyl ether,
• a copolymer composed of 30-60% by weight ethyl acrylate and 40-70% by weight methacrylic acid,
• a copolymer composed of 40-60% by weight ethyl acrylate, 30-50% by weight methacrylic acid, 5-15% by weight acrylic acid, and 0.01-1.0% by weight pentaerythritol triallyl ether,
• a terpolymer composed of 60-80% by weight tert-butyl acrylate, 5-20% by weight ethyl acrylate and 10-30% by weight methacrylic acid,
• a copolymer composed of 40-65% by weight vinylpyrrolidone, 25-35% by weight methacrylamide, 5-20% by weight vinylimidazole and 5-20% by weight quaternary (methylated) vinylimidazole, and
• a copolymer composed of 50-70% by weight vinylpyrrolidone, 20-40% by weight methacrylamide and 1-10% by weight vinylimidazole.

8. The cosmetic, non-therapeutic use as claimed in any of claims 1 to 7, wherein a cosmetic product is used comprising the polymer and in addition other known cosmetic ingredients, and wherein the polymer is present in the product at a concentration of 0.01 to 5% by weight, preferably 0.05 to 2% by weight, preferably 0.1 to 1.5% by weight.

9. The cosmetic, non-therapeutic use as claimed in claim 8, wherein the cosmetic product, in addition to the polymer, comprises at least water as the additional other known cosmetic ingredient, and wherein the product comprises at least 20% by weight, preferably at least 50% by weight, preferably at least 99.9% by weight water, and wherein the polymer is present fully dispersed, suspended, dissolved or swollen in water at 20°C.

10. The cosmetic, non-therapeutic use as claimed in any of claims 1 to 9, wherein a cosmetic product is used comprising the polymer and in addition other known cosmetic ingredients, and wherein the cosmetic product is one that serves to protect, to care for and/or to cleanse the skin, or is one that serves to care for and/or to cleanse the hair.

11. The cosmetic, non-therapeutic use as claimed in any of claims 1 to 9, wherein a cosmetic product is used comprising the polymer and in addition other known cosmetic ingredients, and wherein the cosmetic product is selected from the group consisting of an aqueous polymer solution (i.e. a hydrogel), an emulsifier-free emulsion (i.e. a gel cream), an emulsion such as a cream or a lotion.

12. A cosmetic, non-therapeutic method for invoking an anti-pollution effect on human skin or on human hair, particularly to protect human skin or human hair from particulate matter, especially fine particulates, comprising the application of the polymer as defined in any of the preceding patent claims, or comprising the application of a cosmetic product comprising the polymer as defined in any of the preceding patent claims and in addition comprising other known cosmetic ingredients, to such human skin or such human hair that require an anti-pollution effect, particularly protection from particulate matter, especially protection from fine particulates.

## Revendications

1. Utilisation cosmétique, non thérapeutique d'un polymère ou d'un produit cosmétique contenant ce polymère et de plus d'autres ingrédients cosmétiques connus, pour l'induction d'un effet anti-pollution pour une peau humaine ou pour des cheveux humains, en particulier pour la protection d'une peau humaine ou de cheveux humains contre la poussière, en particulier contre la poussière fine,
le polymère ayant une solubilité dans l'eau d'au moins 0,01 g de polymère par 100 g d'eau à 20 °C à au moins une valeur de pH dans la plage comprise entre 4 et 9,
et le polymère étant choisi dans le groupe constitué par
• un copolymère d'acide méthacrylique et d'acrylate d'éthyle, qui contient éventuellement en outre des motifs répétitifs qui sont issus d'au moins un monomère choisi dans le groupe constitué par l'acide acrylique, le méthacrylamide, un acrylate d'alkyle comportant 2 à 30 atomes de C dans le groupe alkyle, un méthacrylate alkyle comportant 2 à 30 atomes de C dans le groupe alkyle, un ester d'acide méthacrylique avec un alcool gras en C12 à C30, l'alcool gras étant éthoxylé avec 1 à 100 motifs d' oxyde d'éthylène, et un monomère qui contient au moins deux doubles liaisons éthyléniquement insaturées polymérisables, de sorte que celles-ci puissent avoir pour effet une ramification ou une réticulation, et
• un copolymère de vinylpyrrolidone avec au moins un monomère polymérisable éthyléniquement insaturé supplémentaire, la proportion de la vinylpyrrolidone dans ce copolymère étant d'au plus 90 % en poids, préférablement d'au plus 80 % en poids, en particulier d'au plus 70 % en poids,
dans le cas où le polymère contient des motifs répétitifs issus d'acide acrylique ou d'acide méthacrylique, ces motifs répétitifs sont présents en tant qu'acides libres ou en tant que sels, lorsqu'ils sont présents en tant que sels, les sels étant préférablement des sels d'alcali, en particulier des sels de sodium.

2. Utilisation cosmétique, non thérapeutique selon la revendication 1, le monomère qui contient au moins deux doubles liaisons éthyléniquement insaturées polymérisables étant l'éther de triallyle de pentaérythritol.

3. Utilisation cosmétique, non thérapeutique selon la revendication 1, le polymère étant un copolymère de vinylpyrrolidone avec au moins un monomère polymérisable éthyléniquement insaturé supplémentaire, la proportion de la vinylpyrrolidone dans ce copolymère étant d'au plus 90 % en poids, préférablement d'au plus 80 % en poids, en particulier d'au plus 70 % en poids, et l'au moins un monomère polymérisable éthyléniquement insaturé supplémentaire étant choisi dans le groupe constitué par le vinylimidazole, le méthyl-vinylimidazolium, conjointement avec un quelconque anion, l'acrylamide et le méthacrylamide.

4. Utilisation cosmétique, non thérapeutique selon la revendication 3, l'anion étant choisi dans le groupe constitué par chlorure et (H₃C-O-SO₃).

5. Utilisation cosmétique, non thérapeutique selon l'une quelconque des revendications 1 à 4, le polymère ayant une température de transition vitreuse d'au moins 70 °C.

6. Utilisation cosmétique, non thérapeutique selon l'une quelconque des revendications 1 à 5, le polymère ayant une masse molaire moyenne (Mw moyenne en poids) d'au moins 100 000 g/mole.

7. Utilisation cosmétique, non thérapeutique selon l'une quelconque des revendications 1 à 6, le polymère étant choisi dans le groupe constitué par
• un copolymère composé de 30 à 60 % en poids d'acrylate d'éthyle, 30 à 60 % en poids d'acide méthacrylique, 5 à 30 % en poids de méthacrylamide, 0,1 à 10 % en poids de méthacrylate d'alkyle en C18-(EO)-25 et 0,01 à 1,0 % en poids d'éther de triallyle de pentaérythritol,
• un copolymère composé de 30 à 60 % en poids d'acrylate d'éthyle et 40 à 70 % en poids d'acide méthacrylique,
• un copolymère composé de 40 à 60 % en poids d'acrylate d'éthyle, 30 à 50 % en poids d'acide méthacrylique, 5 à 15 % en poids d'acide acrylique et 0,01 à 1,0 % en poids d'éther de triallyle de pentaérythritol,
• un terpolymère de 60 à 80 % en poids d'acrylate de tert-butyle, 5 à 20 % en poids d'acrylate d'éthyle et 10 à 30 % en poids d'acide méthacrylique,
• un copolymère de 40 à 65 % en poids de vinylpyrrolidone, 25 à 35 % en poids de méthacrylamide, 5 à 20 % en poids de vinylimidazole et 5 à 20 % en poids de vinylimidazole quaternaire (méthylé), et
• un copolymère de 50 à 70 % en poids de vinylpyrrolidone, 20 à 40 % en poids de méthacrylamide et 1 à 10 % en poids de vinylimidazole.

8. Utilisation cosmétique, non thérapeutique selon l'une quelconque des revendications 1 à 7, un produit cosmétique contenant le polymère et de plus d'autres ingrédients cosmétiques connus étant utilisé, et le polymère étant contenu dans le produit en une concentration de 0,01 à 5 % en poids, préférablement de 0,05 à 2 % en poids, préférablement de 0,1 à 1,5 % en poids.

9. Utilisation cosmétique, non thérapeutique selon la revendication 8, le produit cosmétique contenant outre le polymère au moins de l'eau en tant qu'autre ingrédient cosmétique connu supplémentaire, et le produit contenant au moins 20 % en poids, préférablement au moins 50 % en poids, préférablement au moins 99,9 % en poids, d'eau, et le polymère étant présent à 20 °C totalement dispersé, mis en suspension, dissous ou gonflé dans l'eau.

10. Utilisation cosmétique, non thérapeutique selon l'une quelconque des revendications 1 à 9, un produit cosmétique contenant le polymère et de plus d'autres ingrédients cosmétiques connus étant utilisé, et le produit cosmétique en étant un qui sert pour la protection, pour l'entretien et/ou le nettoyage de la peau, ou en étant un qui sert pour l'entretien et/ou le nettoyage des cheveux.

11. Utilisation cosmétique, non thérapeutique selon l'une quelconque des revendications 1 à 9, un produit cosmétique contenant le polymère et de plus d'autres ingrédients cosmétiques connus étant utilisé, et le produit cosmétique étant choisi dans le groupe constitué par une solution aqueuse de polymère (c'est-à-dire un hydrogel), une émulsion exempte d'émulsifiant (c'est-à-dire une gel-crème), une émulsion comme par ex. une crème ou une lotion.

12. Procédé cosmétique, non thérapeutique d'induction d'un effet anti-pollution pour une peau humaine ou des cheveux humains, en particulier de protection d'une peau humaine ou de cheveux humains contre la poussière, en particulier contre la poussière fine, comprenant l'application du polymère comme défini dans l'une quelconque des revendications de brevets précédentes, ou comprenant l'application d'un produit cosmétique contenant le polymère comme défini dans l'une quelconque des revendications de brevets précédentes et de plus contenant d'autres ingrédients cosmétiques connus, sur une telle peau humaine ou sur de tels cheveux humains, qui ont besoin d'un effet anti-pollution, en particulier la protection contre la poussière, en particulier la protection contre la poussière fine.
